# EUROPEAN PATENT APPLICATION

(11) **EP 2 110 081 A1**
(43) Date of publication of application: **21.10.2009**
(21) Application number: 09251087.4
(22) Date of filing: 14.04.2009
(51) Int. Cl.: A61B 17/06

(54) **Apparatus for the joining of tissue having integral penetrating end**

(30) Priority: 15.04.2008 US 44968 P; 27.03.2009 US 412613
(71) Applicant: Tyco Healthcare Group LP, North Haven, CT 06473 (US)
(72) Inventor: Kennedy, John J, Guildford, CT 06437 (US); Stopek, Joshua, Yalesville, CT 03649 (US)
(74) Representative: Soames, Candida Jane

(57) **Abstract**

The present disclosure relates to an apparatus for joining tissue in surgical applications and/or incision repair, and to methods for making the same. The apparatus includes an elongated member formed of a biocompatible material, and a rigidifying agent associated with a distal end portion of the elongated member, wherein the rigidifying agent increases the rigidity of the distal end portion such that the distal end portion is mechanically reconfigurable to define a penetrating end integrally formed with the elongated member.

## Description

### CROSS-REFERENCE TO RELATED APPLICATIONS

This application claims the benefit of U.S. Provisional Application No. 61/044,968, filed on April 15, 2008.

### BACKGROUND

### 1. Technical Field

The present disclosure relates to an apparatus for the joining of tissue in surgical applications, and to methods for making the same. More particularly, the present disclosure relates to a surgical suturing apparatus for use during incision or wound repair, and methods of making the same.

### 2. Background of the Related Art

The structures and methods facilitating the attachment of a suture, or ligament, to a needle are well known in the art. Such needle-suture combinations are provided for a wide variety of monofilament and multifilament suture materials, in both the absorbable and non-absorbable varieties. These suture materials may be formed, for example, from catgut, silk, nylon, polyesters, polypropylene, steel, or absorbable synthetic materials such as polymers and copolymers of glycolic acid, lactic acid, dioxanone, caprolactone, and trimethylene carbonate.

Needle-suture combinations fall into two general classes, i.e. standard needle attachment, in which the suture is securely attached to the needle and is not intended to be separable therefrom except by cutting or severing, and removable or detachable needle attachment, in which the needle is separable from the suture in response to a force exerted by the clinician.

Various methods for both standard and detachable needle attachment are known in the art, one of the most conventional being the coupling of the shank end of a needle with the suture. However, when coupling a needle and suture in this manner, the possibility of inadvertent detachment of the needle from the suture exists. To address this potentiality, methods of integrally or monolithically forming the needle with the suture have been developed.

U.S. Patent Nos. 5,531,761; 7,056,331; and 5,342,376 each relate to the use of a suture having a body that is integrally formed with a sharpened distal end, and suggest the inclusion of a material that is sufficiently rigid to facilitate the penetration of tissue with the distal end, such as a polymeric or co-polymeric materials.

U.S. Patent No. 4,602,636 teaches the use of a wire suture formed of stainless steel or cobalt chromium alloys, for example, that includes a work hardened needle-like tip that is harder and stronger than the remainder of the suture.

Each of the methods for integrally forming a needle with a suture discussed above has associated disadvantages and difficulties which may be encountered during use or production. Accordingly, a need exists in the art for an improved surgical suturing apparatus, and a method of making the same, which overcomes these deficiencies.

### SUMMARY

In one aspect of the present disclosure, an apparatus for the joining of tissue is disclosed that includes an elongated member and a rigidifying agent. The rigidifying agent is associated with a distal end portion of the elongated member and increases the rigidity thereof in order to render the distal end portion mechanically reconfigurable such that a penetrating end integrally formed with the elongated member can be defined. In one embodiment, the penetrating end is configured to facilitate insertion of the apparatus into tissue.

In certain embodiments, the elongated member is formed of a biocompatible material, which may be bioabsorbable. In one embodiment, the elongated member is composed of a plurality of filaments arranged so as to define a plurality of interstices therebetween. In these embodiments, the rigidifying agent is at least partially disposed within the interstices of the distal end portion such that the rigidifying agent is maintained in the distal end portion in an amount substantially within the range of approximately 1% of the weight of the elongated member to approximately 150% of the weight of the elongated member. For example, the rigidifying agent may be maintained in the distal end portion at up to 20% of the weight of the elongated member. The rigidifying agent may be any biocompatible thermoplastic polymer, including but not limited to isocyanates, cyanoacrylates, cyanoacrylate monomers, photo polymerizable monomers, thermo polymerizable monomers, gamma-radiation polymerizable monomers, e.g., ultraviolet polymerizable monomers, and chemical polymerizable monomers.

The present disclosure contemplates that the elongated member may include a plurality of barbs. Alternatively, or additionally, the distal end portion of the elongated member may define a sharp tip, and in some embodiments, may be configured as a needle having, for example, an arcuate configuration or a distally tapered configuration.

The elongated member may include a weakened portion located proximally of the distal end portion to facilitate selective detachment thereof.

In another aspect of the present disclosure, a method of manufacturing an apparatus for the joining of tissue is disclosed. The method includes the steps of providing an elongated member formed of a biocompatible material, associating a rigidifying agent with a distal end portion thereof, increasing the rigidity of the rigidifying agent to thereby increase the rigidity of the distal end portion, and mechanically reconfiguring the distal end portion to define a penetrating end that is integrally formed with the elongated member.

The step of providing an elongated member may include providing an elongated material that is formed of a plurality of filaments defining interstices therebetween for retaining the rigidifying agent, in which case the step of associating the rigidifying agent with the distal end portion may include impregnation of the distal end portion with the rigidifying agent to thereby dispose the rigidifying agent within the interstices of the distal end portion.

The step of mechanically reconfiguring the distal end portion may include subjecting the distal end portion to heat and pressure to thereby form the aforementioned penetrating end.

The step of associating a rigidifying agent with the distal end portion may include the introduction of a biocompatible thermoplastic polymer. Examples of suitable biocompatible thermoplastic polymers include, but are not limited to cyanoacrylate monomers, PMMA (polymethyl methacrylate), PLGA (polylactic-co-glycolic acid), and polyhydroxyacetic acid.

These and other features of the apparatus disclosed herein, and methods of making the same, will become more readily apparent to those skilled in the art from the following detailed description of various embodiments of the present disclosure.

### BRIEF DESCRIPTION OF THE DRAWINGS

Various embodiments of the present disclosure are described hereinbelow with references to the drawings, wherein:

**FIG. 1** is a side, perspective view of an exemplary apparatus for the joining of tissue;

**FIG. 2** is a side, perspective view of one embodiment of the apparatus seen in **FIG. 1** including a plurality of barbs formed on a distal portion thereof;

**FIG. 3** is a side, perspective view of another embodiment of the apparatus seen in **FIG. 1** including a weakened portion;

**FIG. 4** is a side, perspective view of yet another embodiment of the apparatus seen in FIG. 1, in which the apparatus includes an elongated member comprised of a plurality of fibers;

**FIG. 5** is a side, perspective view of an alternate embodiment of the apparatus shown in **FIG. 4**, in which the plurality of fibers are configured in a braid;

**FIG. 6** is an enlarged view of the area of detail indicated in FIG. 5;

**FIG. 7** is a top, perspective view of a mold comprising first and second mold portions, shown in spaced apart relation, for use in a method of manufacturing the apparatus seen in **FIG. 1****;** and

**FIG. 8** is a top, perspective view of the first and second mold portions seen in **FIG. 7** shown in juxtaposed arrangement.

### DETAILED DESCRIPTION OF VARIOUS EMBODIMENTS

In the drawings, and in the description which follows, in which like references characters identify similar or identical elements, the term "proximal" should be understood as referring to the end of the apparatus that is closest to the clinician during use, whereas the term "distal" should be understood as referring to the end of the apparatus that is furthest from the clinician during use. In addition, use of the term "tissue" herein should be understood as referring to any bodily tissue including, but not limited to, skin, fascia, ligaments, tendons, muscle, and bone.

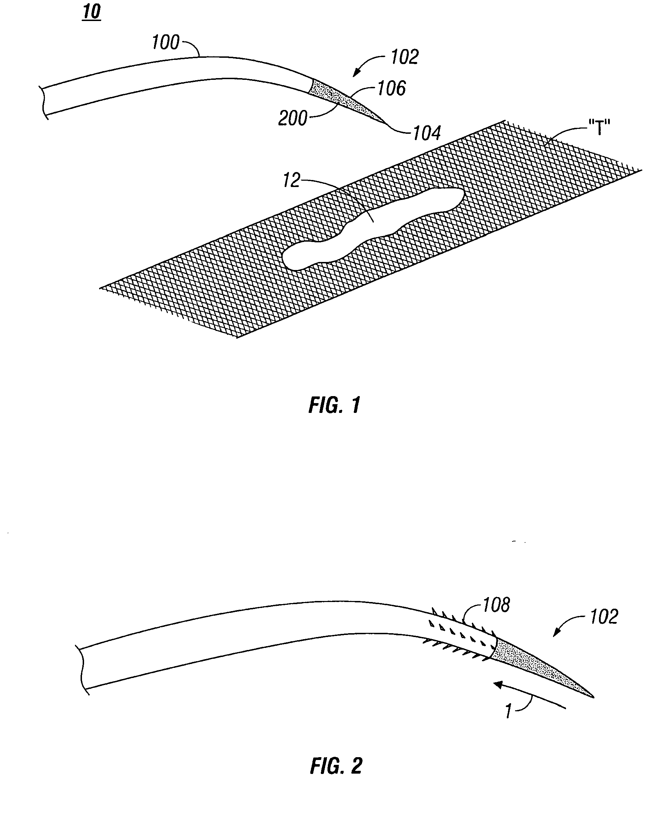

With reference now to **FIG. 1****,** an apparatus **10** in accordance with the principles of the present disclosure is illustrated. The apparatus **10** includes an elongated member **100** having a distal end portion **102** that includes a penetrating end **104** formed integrally therewith. The penetrating end **104** may define a sharp tip, as seen in **FIG. 1****,** for example, thereby enabling the apparatus **10** to penetrate tissue. In some embodiments, the penetrating end **104** may be configured and dimensioned so as to define a needle, or configured similarly in shape to a traditional needle, e.g., a steel needle.

Desirably, the elongated member **100** has a measure of flexibility such that the suturing apparatus **10** can be manipulated by the clinician to join adjacent sections of the tissue **"T"** together. As an illustrative example, the apparatus **10** may be employed to repair or close an incision **12,** wound, or the like formed in the tissue **"T"** using conventional suturing techniques. The elongated member **100** may be any elongated member, e.g. a suture, ligature, or surgical tape, formed from a suitable biocompatible, including but not limited to polypropylene, polyester, nylon, or other polymeric materials. In one embodiment, it is envisioned that the elongated member **100** may be formed of a bioabsorbable material.

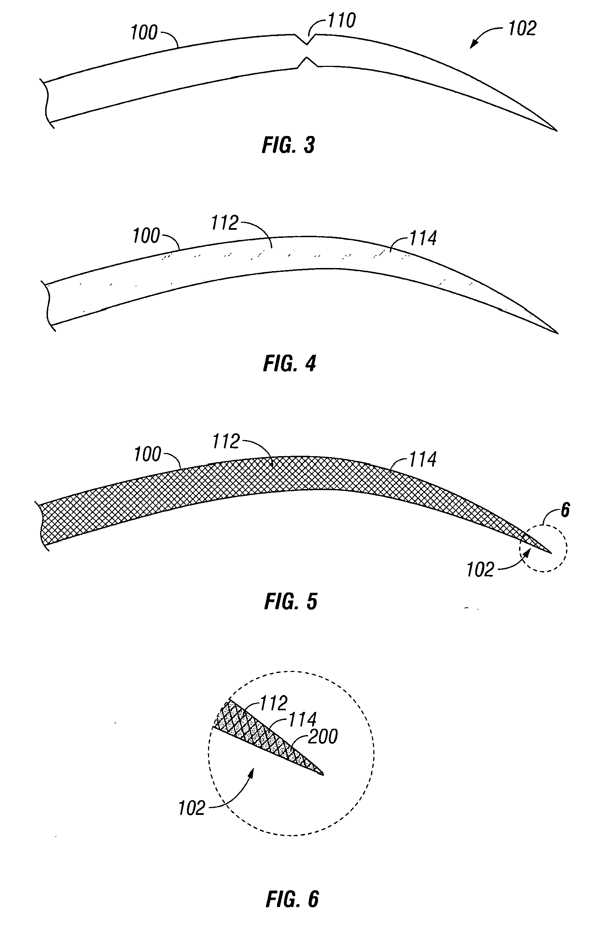

The distal end portion **102,** and the penetrating end **104** formed integrally therewith, may exhibit any configuration that facilitates the penetration of tissue. Accordingly, the penetrating end **104** may be substantially incisive, as shown, or substantially blunt. As seen in **FIG. 1****,** in one embodiment, the distal end portion **102** may be configured as a needle **106.** The needle **106** may exhibit any configuration suitable for the intended purpose of facilitating the passage of the elongated member **100** through the tissue **"T."** To this end, the needle **106** may define an arcuate or linear configuration, and may be tapered such that the surface area thereof decreases distally along its length. As seen in **FIG. 2****,** the distal end portion **102** may include a plurality of barbs **108** formed thereon to inhibit removal, or reversal, of the distal end portion **102** from the tissue **"T" (****FIG.** 1) in the proximal direction indicated by arrow **1.** Additionally, or alternatively, the elongated member **100** may include a weakened portion **110** that is located proximally of the distal end portion **102,** as seen in **FIG. 3****,** such that the clinician may selectively detach the distal end portion **102** from the remainder of the elongated member **100** upon successfully joining the tissue **"T" (****FIG. 1****).**

Referring again to **FIG. 1****,** prior to formation of the penetrating end **104,** a rigidifying agent **200** is associated with the distal end portion **102.** In one embodiment of the present disclosure, the rigidifying agent is a thermoplastic polymer, such as a cyanoacrylate monomer that would polymerize once it has penetrated into or coated the distal end portion **102.** However, the use of other polymers, including but not limited to isocyanates, cyanoacrylates, cyanoacrylate monomers, photo polymerizable monomers, thermo polymerizable monomers, radiation polymerizable monomers, e.g., ultraviolet polymerizable monomers, and chemical polymerizable monomers., or polymerizations, e.g., photo-initiated polymerization, is not beyond the scope of the present disclosure.

The rigidifying agent **200** is adapted to transition from a first, or initial condition, to a second condition upon the application of energy thereto. The energy may be created in any suitable manner, and may be in the form of pressure, heat, or irradiation. Alternatively, the requisite energy may be created using a chemical reaction, e.g., curing. In the initial condition, the rigidifying agent **200** is substantially pliable and/or malleable such that it may be applied to the distal end portion **102.** The rigidifying agent **200** may be applied to the distal end portion **102** in any suitable manner, such as by spraying or dip coating the distal end portion **102.** In the second condition, the rigidifying agent **200** is substantially more rigid, thereby rendering the distal end portion **102** substantially more rigid as well and susceptible to mechanical reconfiguration to thereby define the penetrating end **104,** as discussed in further detail below.

With reference to **FIGS. 4-6****,** in one embodiment, the elongated member **100** is composed of a plurality of filaments **112.** The filaments **112** are arranged to define a plurality of interstices **114** therebetween, and may be arranged in any manner suitable for this intended purpose, including but not limited to braiding, entangling, weaving, or comingling the plurality of filaments **112.** The filaments **112** may be loosely interwoven, as seen in **FIG. 4****,** or alternatively, the filaments may be arranged in a braided configuration, as seen in **FIG. 5****.** In the embodiment of **FIGS. 4-6****,** upon the association of the rigidifying agent **200** with the elongated member **100,** at least a portion of the rigidifying agent **200** is disposed within the interstices of the distal end portion **102.** The rigidifying agent may be maintained within the distal end portion in an amount substantially within the range of approximately 1% of the weight of the elongated member **100** to approximately 150% of the weight of the elongated member **100.**

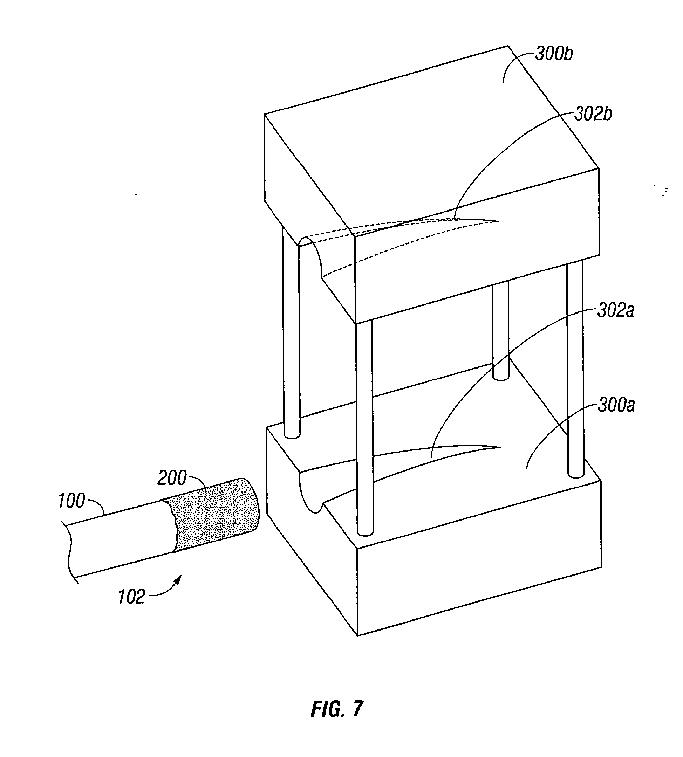

Referring now to **FIGS. 1****,** **7****,** and **8****,** a method of manufacturing the apparatus **10** discussed above will be described. Initially, the elongated member **100** is provided and the rigidifying agent **200** is associated with the distal end portion **102** thereof. It should be noted that the elongated member **100** illustrated in **FIG. 7** does not yet include the penetrating end **104** depicted in **FIG. 1****,** as the penetrating end **104** is created during the process described below.

As previously discussed, the rigidifying agent **200** is in a substantially pliable and/or malleable condition during application to the distal portion **102** of the elongated member **100.** Subsequently, however, the rigidifying agent **200** is caused to transition to the second condition, during which the rigidifying agent **200** experiences a substantial increase in rigidity concomitantly with the distal end portion **102.** Upon realizing sufficient rigidity, the distal end portion **102** is mechanically reconfigured to define the penetrating end **104.**

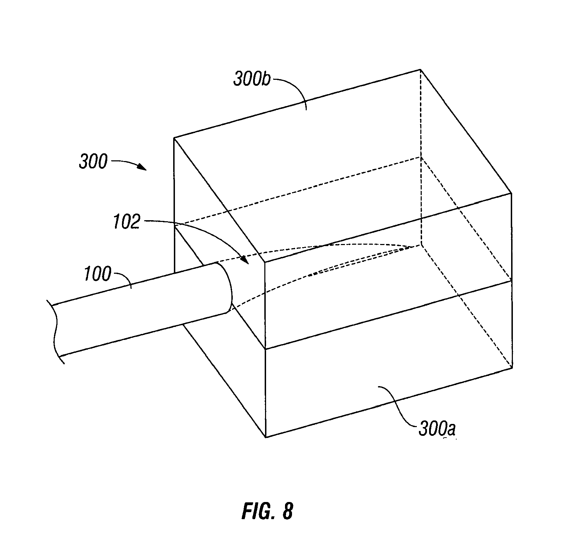

Referring still to **FIGS. 1****,** **7****,** and **8****,** in one embodiment of the aforedescribed method, the rigidifying agent **200,** and consequently, the distal end portion **102,** are caused to rigidify through the application of heat and/or pressure thereto. One suitable manner in which the necessary heat and/or pressure may be created and applied is through the employ of compression molding. During this process, subsequent to the application of the rigidifying agent **200** to the distal end portion **102,** the distal end portion **102** is placed within an open first mold portion **300_{A} (****FIG. 7****)** having a first cavity **302_{A}** formed therein that defines a configuration corresponding to that which is desired for the penetrating end **104 (****FIG. 1****)** and the remainder of the distal end portion **102.** Thereafter, a second mold portion **300_{B}** with a second cavity **302_{B}** formed therein is brought into juxtaposed arrangement with the first mold portion **300_{A},** as seen in **FIG. 8****,** thereby applying a controllable level of pressure and/or heat to the distal end portion **102.** The malleability of the rigidifying agent **200** in its first condition allows the distal end portion **102** to be reconfigured, e.g. reshaped, such that that distal end portion **102** exhibits the configuration collectively defined by the respective first and second cavities **302_{A}, 302_{B}** of the first and second mold portions **300_{A}, 300_{B}.** The pressure and/or heat applied to the distal end portion **102** transitions the rigidifying agent **200** from its first condition to its second condition, which facilitates the creation of an elongated member **100** having a substantially rigid penetrating end **104** formed integrally therewith.

In general, the second cavity **302_{B}** will define a configuration that is substantially similar to that of the first cavity **302_{A},** although a mold portion **300** including respective first and second mold cavities **302_{A}, 302_{B}** that are dissimilar is not beyond the scope of the present disclosure. It should be noted that the respective first and second cavities **302_{A}, 302_{B}** may be configured to yield an elongated member **100** having a distal end portion **102** with an arcuate, tapered configuration, as discussed above with respect to **FIG. 1****,** or a configuration that includes a plurality of barbs **108,** as discussed above with respect to **FIG. 2****.** Other methods which may be used to reconfigure the distal end portion **102** to define the penetrating end **104** are also within the purview of those skilled in the art, and include, but are not limited to the use of ultrasonic energy, blades, molds, and dies.

During the compression molding process, the mold portion **300** may be heated either prior, or subsequent, to the juxtaposition of the respective first and second mold portions **300_{A}, 300_{B},** such that a controllable level of heat may be applied to the distal end portion **102.** The application of heat may act to further facilitate the reconfiguration of the distal end portion **102** and/or the transition of the rigidifying agent **200** from the first condition to the second condition.

For the purposes of discussion, in one embodiment, it is contemplated that the elongated member **100** may be a Polysorb^{™} multifilament absorbable suture that is treated with octyl cyanoacrylate as the rigidifying agent **200.** The distal end portion **102** of the Polysorb^{™} suture is dipped into the octyl cyanoacrylate such that the octyl cyanoacrylate is disposed within the interstices **114 (****FIG. 3****)** defined between the plurality of filaments **112** of the Polysorb^{™} suture and embedded within the distal end portion **102** thereof. The octyl cyanoacrylate is then allowed to cure, during which time the rigidity of the distal end portion **102** increases. Either during the curing process, or subsequently thereafter, the distal end portion **102** is placed into a mold, e.g., between the respective first and second mold portions **302_{A}, 302_{B} (****FIGS. 7**,**8)**, to form the penetrating end **104** into a needle-like shape, for example.

Although the method of manufacture disclosed herein and illustrated in **FIGS. 7** and **8** has been discussed with respect to the elongated member **100** of the apparatus **10 (****FIG. 1****),** in alternative embodiments of the present disclosure, it is envisioned that the method of manufacture may be employed in connection with various other structures. For example, the presently disclosed method of manufacture may be used in the fabrication of a self-gripping surgical mesh, such as the Parietex ProGrip^{™}, which is distributed commercially by Covidien, 15 Hampshire Street, Mansfield, MA, USA, for use during open inguinal hernia repair, and discussed in U.S. Patent Application Serial No. 12/032,750, filed on February 18, 2008, the entire contents of which are incorporated by reference herein.

The above description, disclosure, and figures should not be construed as limiting, but merely as exemplary of particular embodiments. It is to be understood, therefore, that the disclosure is not limited to the precise embodiments described, and that various other changes and modifications may be effected by one skilled in the art without departing from the scope or spirit of the disclosure. Additionally, those skilled in the art will appreciate that the elements and features illustrated or described in connection with one embodiment can be combined with those of another, and that such modifications and variations are also intended to be included within the scope of the present disclosure.

## Claims

1. An apparatus for the joining of tissue, comprising:
an elongated member formed of a biocompatible material; and
a rigidifying agent integrated into a first end portion of the elongated member, wherein the rigidifying agent increases the rigidity of the first end portion such that the first end portion such that the first end portion defines a penetrating end integrally formed with the elongated member.

2. The apparatus of claim 1, wherein the penetrating end is configured to facilitate insertion of the apparatus into tissue; or the apparatus of claim 1, wherein the elongated member is formed of a bioabsorbable material, or the apparatus of claim 1, wherein the elongated member comprises a plurality of filaments.

3. The apparatus of claim 2, wherein the plurality of filaments are arranged so as to define interstices therebetween, the rigidifying agent being at least partially disposed within the interstices of the first end portion.

4. The apparatus of claim 1, wherein the rigidifying agent is maintained in the first end portion in an amount substantially within the range of approximately 1% of a weight of the elongated member to approximately 150% of the weight of the elongated member.

5. The apparatus of claim 4, wherein the rigidifying agent is maintained in the first end portion at up to 20% of the weight of the elongated member.

6. The apparatus of claim 1, wherein the elongated member includes a plurality of barbs; or the apparatus of claim 1, wherein the elongated member includes a weakened portion located proximally of the first end portion to facilitate selective detachment thereof; or the apparatus of claim 1, wherein the penetrating end defines a sharp tip; or the apparatus of claim 1, wherein the first end portion is configured as a needle.

7. The apparatus of claim 1, wherein the rigidifying agent is a biocompatible thermoplastic polymer.

8. The apparatus of claim 7 wherein the rigidifying agent is selected from the group consisting of cyanoacrylate monomers, isocyanates, silicones, and ultraviolet polymerizable polyacrylates.

9. An apparatus for the joining of tissue, comprising:
an elongated member formed of a biocompatible material; and
a rigidifying agent associated with a distal end portion of the elongated member, wherein the rigidifying agent increases the rigidity of the distal end portion such that the distal end portion is mechanically reconfigurable to define a penetrating end integrally formed with the elongated member.

10. A method of manufacturing an apparatus for the joining of tissue, comprising the steps of:
providing an elongated member formed of a biocompatible material;
associating a rigidifying agent with a distal end portion of the elongated member;
increasing the rigidity of the rigidifying agent to thereby increase the rigidity of the distal end portion; and
mechanically reconfiguring the distal end portion to define a penetrating end integrally formed with the elongated member.

11. The method of claim 10, wherein the step of providing an elongated member includes providing an elongated material formed of a plurality of filaments defining interstices therebetween for retaining the rigidifying agent.

12. The method of claim 11 wherein the step of associating the rigidifying agent with the distal end portion includes impregnating the distal end portion with the rigidifying agent to thereby dispose the rigidifying agent within the interstices of the distal end portion.

13. The method of claim 12 wherein the step of mechanically reconfiguring the distal end portion includes subjecting the distal end portion to heat and pressure to thereby form the penetrating end.

14. The method of claim 12, wherein the step of associating a rigidifying agent with the distal end portion includes associating a biocompatible thermoplastic polymer with the distal end portion.

15. The method of claim 14 wherein the step of associating a rigidifying agent with the distal end portion includes associating a polymer with the distal end portion that is selected from the group consisting of cyanoacrylate monomers, isocyanates, silicones, and ultraviolet polymerizable polyacrylates.
